# EUROPÄISCHE PATENTANMELDUNG

(11) **EP 1 300 133 A2**
(43) Veröffentlichungstag der Anmeldung: **09.04.2003**
(21) Anmeldenummer: 02021428.4
(22) Anmeldetag: 25.09.2002
(51) Int. Cl.: A61K 7/13, D06P 3/14

(54) **Mittel zum Färben von keratinhaltigen Fasern**

(30) Priorität: 04.10.2001 DE 10148850
(71) Anmelder: Henkel Kommanditgesellschaft auf Aktien, 40589 Düsseldorf-Holthausen (DE)
(72) Erfinder: Möller, Hinrich, Dr., 40789 Monheim (DE); Oberkobusch, Doris, Dr., 40591 Düsseldorf (DE); Höffkes, Horst, Dr., 40595 Düsseldorf (DE)

(57) **Zusammenfassung**

Es wird ein Mittel zum Färben von keratinhaltigen Fasern, insbesondere menschlichen Haaren, beansprucht, enthaltend mindestens eine Kombination aus Aldehyden bzw. Ketonen mit der Formel I, 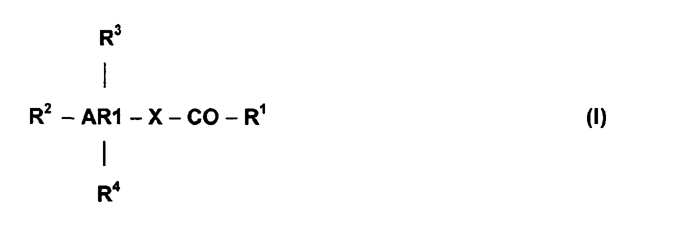 in der bedeuten
- AR1 Benzol, Naphthalin, Pyridin, Furan, Chinolin, Isochinolin, Chinolizin, Pyrimidin, Pyrrol, Thiophen, Indol, Indan, Imidazol, Benzimidazol, Benzothiazol, Indazol, Benzoxazol, Carbazol, Chinoxalin, Chinazolin, Cinnolin, Acenaphthen, Fluoren, Biphenyl, Diphenylmethan, Benzophenon, Diphenylether, Azobenzol, Diphenylamin, Stilben sowie deren teilweise oder vollständig hydrierte Derivate,
- R¹ ein Wasserstoffatom, eine C₁₋₄-Alkyl-, C₂₋₄-Alkenyl-, eine ggf. substituierte Aryl- oder Heteroarylgruppe,
- R², R³ und R⁴ unabhängig voneinander ein Wasserstoff-, ein Halogenatom, eine C₁₋₄-Alkyl-, C₁₋₄-Alkoxy-, C₁₋₄-Acyl-, eine C₁₋₄-Hydroxyalkyl-, eine Hydroxy-C₁₋₄alkoxy-, eine Hydroxy-, eine Sulfo-, eine Carboxy-, eine Nitro-, eine Morpholino-, eine Pyrrolidino-, eine Amino-, C₁₋₄-Acylamino-, eine Ammonio- oder eine 1-lmidazoli(ni)ogruppe, wobei die letzten 4 Gruppen mit C₁₋₄-Alkyl-, C₁₋₄-Carboxyalkyl-, C₁₋₄-Hydroxyalkyl-, C₂₋₆-Oligohydroxyalkyl-, C₂₋₄-Alkenylgruppen, mit ggf. substituierten Benzylgruppen, mit Sulfo-(C₁₋₄)-alkyl- oder Heterozyklus-(C₁₋₄)-alkylgruppen substituiert sein können,
und auch zwei der Gruppen ausgewählt aus R², R³, R⁴ und -X-CO-R¹ miteinander einen ankondensierten gesättigten oder ungesättigten, carbozyklischen oder heterozyklischen, gegebenenfalls substituierten 5-, 6- oder 7-Ring, der ebenfalls einen ankondensierten aromatischen, ggf. substituierten Ring tragen kann, sein können, wobei das System AR1 in Abhängigkeit von der Größe des Ringes weitere Substituenten tragen kann, die unabhängig voneinander für die gleichen Gruppen stehen können wie R², R³ und R⁴, und
- X eine direkte Bindung, eine Carbonylgruppe, eine gegebenenfalls substituierte Methylen, Vinylen-, 1,3-Butadien-1,4-diyl oder Phenylengruppe,
und/oder deren funktionellen Carbonylderivaten und/oder physiologisch verträglichen Salzen
und Nitro-amino-Verbindungen mit der Formel II, 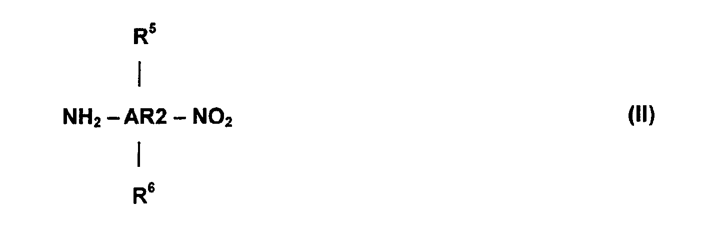 in der bedeuten
- AR2 Benzol, Naphthalin, Pyridin, Furan, Chinolin, Isochinolin, Pyrimidin, Pyrrol, Thiophen, Indol, Benzimidazol, Benzothiazol, Indazol, Benzoxazol, Chinoxalin, Chinazolin, Cinnolin, Acenaphthen, Fluoren, Biphenyl, Diphenylmethan, Benzophenon, Azobenzol, (Naphthyl)phenyldiazen, Diphenylamin, Stilben oder Benzylidenaminobenzol und
- R⁵ und R⁶ unabhängig voneinander ein Wasserstoffatom, Halogenatom, eine C₁₋₄-Alkyl-, C₁₋₄-Alkoxy-, C₁₋₄-Hydroxyalkoxy-, Hydroxy-, Nitro-, Carboxy-, C₁₋₄-Alkoxycarbonyl-, Sulfo-, Sulfamoyl-, eine Aryl- oder Heteroarylgruppe, Arylazogruppe oder eine Aminogruppe, die durch eine oder mehrere C₁₋₄-Alkyloder C₁₋₄-Hydroxyalkylgruppen substituiert sein kann,
wobei das System AR2 in Abhängigkeit von der Größe des Ringes weitere Substituenten tragen kann, die unabhängig voneinander für die gleichen Gruppen stehen können wie R⁵ und R⁶, und zwei der Reste aus R⁵, R⁶ und den ggf. vorhandenen weiteren Resten einen ankondensierten gesättigten oder ungesättigten, carbozyklischen oder heterozyklischen gegebenenfalls substituierten 5-, 6- oder 7-Ring, der ebenfalls einen ankondensierten aromatischen, ggf. substituierten Ring tragen kann, bilden können,
und/oder deren physiologisch verträglichen Salzen,
und/oder Reaktionsprodukte aus den Verbindungen mit der Formel 1 und der Formel II.

## Beschreibung

Die Erfindung betrifft ein Mittel zum Färben von keratinhaltigen Fasern, insbesondere menschlichen Haaren, das eine Kombination aus Aldehyden bzw. Ketonen mit der Formel I und Nitro-amino-Verbindungen mit der Formel II enthält, die Verwendung dieser Kombination als färbende Komponente in Haarfärbemitteln sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2'-hydroxyethyl)-p-phenylendiamin, 2-(2',5'-Diaminophenyl)-ethanol, 2-(2',5'-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2'-hydroxyethylamino)-anisol (Lehmanns Blau), 1-Phenyl-3-methyl-pyrazol-5-on, 2,4-Dichlor-3-aminophenol, 1,3-Bis-(2',4'-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Die Verwendung der unten näher beschriebenen Kombination aus Aldehyden bzw. Ketonen und Nitro-amino-Verbindungen zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die Kombination aus Aldehyden beziehungsweise Ketonen der Formel I und Nitro-amino-Verbindungen der Formel II sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agenzien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend eine Kombination aus Aldehyden bzw. Ketonen mit der Formel I, 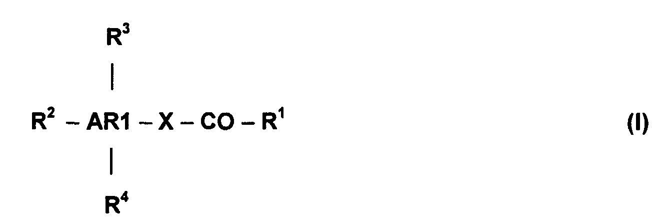 in der bedeuten
- AR1 Benzol, Naphthalin, Pyridin, Furan, Chinolin, Isochinolin, Chinolizin, Pyrimidin, Pyrrol, Thiophen, Indol, Indan, Imidazol, Benzimidazol, Benzothiazol, Indazol, Benzoxazol, Carbazol, Chinoxalin, Chinazolin, Cinnolin, Acenaphthen, Fluoren, Biphenyl, Diphenylmethan, Benzophenon, Diphenylether, Azobenzol, Diphenylamin, Stilben sowie deren teilweise oder vollständig hydrierte Derivate,
- R¹ ein Wasserstoffatom, eine C₁₋₄-Alkyl-, C₂₋₄-Alkenyl-, eine ggf. substituierte Aryloder Heteroarylgruppe,
- R², R³ und R⁴ unabhängig voneinander ein Wasserstoff-, ein Halogenatom, eine C₁₋₄-Alkyl-, C₁₋₄-Alkoxy-, C₁₋₄-Acyl-, eine C₁₋₄-Hydroxyalkyl-, eine Hydroxy-C₁₋₄-alkoxy-, eine Hydroxy-, eine Sulfo-, eine Carboxy-, eine Nitro-, eine Morpholino-, eine Pyrrolidino-, eine Amino-, C₁₋₄-Acylamino-, eine Ammonio- oder eine 1-lmidazoli(ni)ogruppe, wobei die letzten 4 Gruppen mit C₁₋₄-Alkyl-, C₁₋₄-Carboxyalkyl-, C₁₋₄-Hydroxyalkyl-, C₂₋₆-Oligohydroxyalkyl-, C₂₋₄-Alkenylgruppen, mit ggf. substituierten Benzylgruppen, mit Sulfo-(C₁₋₄)-alkyl- oder Heterozyklus-(C₁₋₄)-alkylgruppen substituiert sein können, und auch zwei der Gruppen ausgewählt aus R², R³, R⁴ und -X-CO-R¹ miteinander einen ankondensierten gesättigten oder ungesättigten, carbozyklischen oder heterozyklischen, gegebenenfalls substituierten 5-, 6- oder 7-Ring, der ebenfalls einen ankondensierten aromatischen, ggf. substituierten Ring tragen kann, sein können, wobei das System AR1 in Abhängigkeit von der Größe des Ringes weitere Substituenten tragen kann, die unabhängig voneinander für die gleichen Gruppen stehen können wie R², R³ und R⁴, und
- X eine direkte Bindung, eine Carbonylgruppe, eine gegebenenfalls substituierte Methylen, Vinylen-, 1,3-Butadien-1,4-diyl oder Phenylengruppe,
   und/oder deren funktionellen Carbonylderivaten und/oder physiologisch verträglichen Salzen
und Nitro-amino-Verbindungen mit der Formel II, 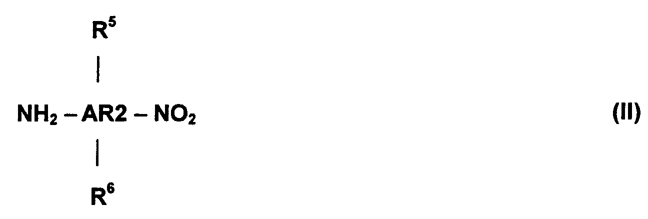 in der bedeuten
- AR2 Benzol, Naphthalin, Pyridin, Furan, Chinolin, Isochinolin, Pyrimidin, Pyrrol, Thiophen, Indol, Benzimidazol, Benzothiazol, Indazol, Benzoxazol, Chinoxalin, Chinazolin, Cinnolin, Acenaphthen, Fluoren, Biphenyl, Diphenylmethan, Benzophenon, Azobenzol, (Naphthyl)phenyldiazen, Diphenylamin, Stilben oder Benzylidenaminobenzol und
- R⁵ und R⁶ unabhängig voneinander ein Wasserstoffatom, Halogenatom, eine C₁₋₄-Alkyl-, C₁₋₄-Alkoxy-, C₁₋₄-Hydroxyalkoxy-, Hydroxy-, Nitro-, Carboxy-, C₁₋₄-Alkoxycarbonyl-, Sulfo-, Sulfamoyl-, eine Aryl- oder Heteroarylgruppe, Arylazogruppe oder eine Aminogruppe, die durch eine oder mehrere C₁₋₄-Alkyl- oder C₁₋₄-Hydroxyalkylgruppen substituiert sein kann,
wobei das System AR2 in Abhängigkeit von der Größe des Ringes weitere Substituenten tragen kann, die unabhängig voneinanderfür die gleichen Gruppen stehen können wie R⁵ und R⁶, und zwei der Reste aus R⁵, R⁶ und den ggf. vorhandenen weiteren Resten einen ankondensierten gesättigten oder ungesättigten, carbozyklischen oder heterozyklischen gegebenenfalls substituierten 5-, 6- oder 7-Ring, der ebenfalls einen ankondensierten aromatischen, ggf. substituierten Ring tragen kann, bilden können,
und/oder deren physiologisch verträglichen Salzen,
und/oder Reaktionsprodukte aus den Verbindungen mit der Formel I und der Formel II.

Beispiele für die als Substituenten im Rahmen dieser Anmeldung genannten C₁₋₄-Alkylreste sind die Gruppen Methyl, Ethyl, Propyl, Isopropyl und Butyl. Ethyl und Methyl sind bevorzugte Alkylreste. Beispiele für bevorzugte C₂₋₄-Alkenylreste sind Vinyl und Allyl. Erfindungsgemäß bevorzugte C₁₋₄-Alkoxyreste sind beispielsweise eine Methoxyoder eine Ethoxygruppe. Weiterhin können als bevorzugte Beispiele für eine C₁₋₄-Monohydroxyalkylgruppe eine Hydroxymethyl-, eine 2-Hydroxyethyl-, eine 2-Hydroxypropyl, eine 3-Hydroxypropyl- oder eine 4-Hydroxybutylgruppe genannt werden. Eine 2-Hydroxyethylgruppe ist besonders bevorzugt. Beispiele für eine bevorzugte C₂₋₆-Oligohydroxyalkylgruppe sind die 1,2-Dihydroxyethylgruppe und die 2,3-Dihydroxypropylgruppe. Eine bevorzugte Hydroxy-C₁₋₄-Alkoxygruppe ist die 2-Hydroxyethoxygruppe. Beispiele für eine Heterozyklus-C₁₋₄-alkylgruppe sind Pyrrolidino-(C₁₋₄)-alkyl, Piperidino-(C₁₋₄)-alkyl, Morpholino-(C₁₋₄)-alkyl, 2-Furyl-(C₁₋₄)-alkyl, 2-Thienyl-(C₁₋₄)-alkyl, 4-Pyridyl-(C₁₋₄)-alkyl, 3-Pyridyl-(C₁₋₄)-alkyl, 2-Pyridyl-(C₁₋₄)-alkyl, Triazolyl-(C₁₋ ₄)-alkyl und 1-Imidazolyl-(C₁₋₄)-alkyl. Beispiele für Halogenatome sind F-, Cl-, oder Br-Atome, wobei Cl-Atome ganz besonders bevorzugt sind. Eine bevorzugte C₁₋₄-Carboxyalkylgruppe ist die 3-Carboxypropylgruppe. Unter funktionellen Carbonylderivaten werden Kondensationsprodukte aus Carbonylverbindunen und Verbindungen mit einer NH₂-Funktionalität verstanden. Beispiele für funktionelle Carbonylderivate sind Oxime und Imine. Die weiteren verwendeten Begriffe leiten sich erfindungsgemäß von den hier gegebenen Definitionen ab.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyloder Acetylcellulose und synthetischer Fasern, wie z. B. Polyamid-, Polyacrylnitril-, Polyurethan- und Polyesterfasern verwendet werden.

Bevorzugt eingesetzte Verbindungen mit der Formel I sind
- 5-(4'-Dimethylaminophenyl)-penta-2,4-dienal, 5-(4'-Diethylaminophenyl)-penta-2,4-dienal, 5-(4'-Methoxyphenyl)-penta-2,4-dienal, 5-(3',4'-Dimethoxyphenyl)-penta-2,4-dienal, 5-(2',4'-Dimethoxyphenyl)-penta-2,4-dienal, 5-(4'-Piperidinophenyl)-penta-2,4-dienal, 5-(4'-Morpholinophenyl)-penta-2,4-dienal, 5-(4'-Pyrrolidinophenyl)-penta-2,4-dienal, 6-(4'-Dimethylaminophenyl)-hexa-2,4-dien-2-on, 6-(4'-Diethylaminophenyl)-hexa-2,4-dien-2-on, 6-(4'-Methoxyphenyl)-hexa-2,4-dien-2-on, 6-(3',4'-Dimethoxyphenyl)-hexa-2,4-dien-2-on, 6-(2',4'-Dimethoxyphenyl)-hexa-2,4-dien-2-on, 6-(4'-Piperidinophenyl)-hexa-2,4-dien-2-on, 6-(4'-Morpholinophenyl)-hexa-2,4-dien-2-on, 6-(4'-Pyrrolidinophenyl)-hexa-2,4-dien-2-on, 5-(4'-Dimethylamino-1'-naphthyl)-penta-2,4-dienal,
- 2-Nitro-piperonal, 5-Nitro-piperonal, 6-Nitro-piperonal, 5-Hydroxy-2-nitro-piperonal, 2-Hydroxy-5-nitro-piperonal, 2-Chlor-6-nitro-piperonal, 5-Chlor-2-nitro-piperonal, 2,6-Dinitro-piperonal,
- 2-Nitro-benzaldehyd, 3-Nitro-benzaldehyd, 4-Nitro-benzaldehyd, 4-Methyl-3-nitrobenzaldehyd, 3-Hydroxy-4-nitro-benzaldehyd, 4-Hydroxy-3-nitro-benzaldehyd, 5-Hydroxy-2-nitro-benzaldehyd, 2-Hydroxy-5-nitro-benzaldehyd, 2-Hydroxy-3-nitrobenzaldehyd, 2-Fluor-3-nitro-benzaldehyd, 3-Methoxy-2-nitro-benzaldehyd, 4-Chlor-3-nitro-benzaldehyd, 2-Chlor-6-nitro-benzaldehyd, 5-Chlor-2-nitro-benzaldehyd, 4-Chlor-2-nitro-benzaldehyd, 2,4-Dinitro-benzaldehyd, 2,6-Dinitro-benzaldehyd, 2-Hydroxy-3-methoxy-5-nitro-benzaldehyd, 4,5-Dimethoxy-2-nitro-benzaldehyd, 5-Nitrovanillin, 3,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd,
- Carbazolaldehyde oder Carbazolketone, insbesondere 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
- 4-Trimethylammoniobenzaldehyd-, 4-Benzyldimethylammoniobenzaldehyd-, 4-Trimethylammoniozimtaldehyd-, 4-Trimethylammonionaphthaldehyd-, 2-Methoxy-4-trimethylammoniobenzaldehyd-, N-(4-Acetylphenyl)-trimethylammonium-, 4-(N,N-Diethyl)-N-methylammonio)-benzaldehyd-, N-(4-Benzoylphenyl)-trimethylammonium-, N-(4-Benzoylphenyl)-N,N-diethylmethylammonium-, N-(4'-Formylphenyl)-N-methylpyrrolidinium-, N-(4'-Formylphenyl)-N-methylpiperidinium-, N-(4-Formylphenyl)-N-methylmorpholinium- N-(4'-Acetylphenyl)-N-methylmorpholinium-, N-(4'-Benzoylphenyl)-N-methylmorpholinium-, 3-Formyl-N-ethyl-N-methylcarbazolium-, 3-Formyl-9,9-dimethylcarbazolium-, 1-(4'-Acetylphenyl)-3-methylimidazolium-, 1-(4'-Acetylphenyl)-3-methyl-2-imidazolinium-, 1-(4'-Benzoylphenyl)-3-methylimidazolium-, 5-Acetyl-1,3-diethyl-2-methylbenzimidazolium-, 5-Trimethylammonio-1-indanon-Salze, insbesondere die Benzolsulfonate, p-Toluolfulfonate, Methansulfonate, Ethansulfonate, Propansulfonate, Perchlorate, Sulfate, Chloride, Bromide, lodide, Tetrachlorzinkate, Methylsulfate, Trifluormethansulfonate, Hexafluorphosphate, Tetrafluorborate,
- Salicylaldehyd, Vanillin, 4-Hydroxy-3-methoxyzimtaldehyd (Coniferylaldehyd), 2,4-Dihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 4-Piperidinobenzaldehyd, 4-Dimethylaminoacetophenon, 4-Hydroxynaphthaldehyd, 4-Dimethylaminonaphthaldehyd, 4-Dimethylaminobenzylidenaceton, 4-Dimethylaminozimtaldehyd, 2-Dimethylaminobenzaldehyd, 2-Chlor-4-dimethylaminobenzaldehyd, 4-Dimethylamino-2-methylbenzaldehyd, trans-4-Diethylamino-zimtaldehyd, 4-(Dibutylamino)-benzaldehyd, 4-Diphenylaminobenzaldehyd, 2,3,6,7-Tetrahydro-1H,5H-benzo[ij]chinolizin-9-carboxaldehyd, 4-Dimethylamino-2-methoxybenzaldehyd, 2,3,6,7-Tetrahydro-8-hydroxy-1 H,5H-benzo[ij] chinolizin-9-carboxaldehyd, 4-(1-Imidazolyl)-benzaldehyd, 2-Morpholinobenzaldehyd, Indol-3-carboxaldehyd, 1-Methylindol-3-carboxaldehyd, N-Ethylcarbazol-3-carboxaldehyd, 2-Formylmethylen-1,3,3-trimethylindolin (Tribasen Aldehyd)
- 1,3-Diacetylbenzol, 1,4-Diacetylbenzol, 1,3,5-Triacetylbenzol, 2-Benzoyl-acetophenon, 2-(4-Methoxybenzoyl)-acetophenon, 2-(2'-Furoyl)-acetophenon, 2-(2'-Pyridoyl)-acetophenon, 2-(3'-Pyridoyl)-acetophenon,
- 1-Phenyl-1,2-propandion, 1-Phenyl-1,2-butandion, 1-(Chlorphenyl)-1,2-propandion, 1-Phenyl-3,3-dimethyl-1,2-butandion, Benzil, Anisil, Salicil, 5,5'-Dibromsalicil, 2,2'-Furyl, 2,2'-Thienyl, 2,2'-, 4,4'-Pyridil, 6,6'-Dimethyl-4,4'-pyridil, 4-Hydroxy-, 4-Methoxy-, 4-Chlor-, 4-Methyl-, 4-Dimethylamino-, 4,4'-Dihydroxy-, -Dimethyl-, -Dibrom-, -Dichlor-, -Bis-dimethylamino-, 2,4-Dihydroxy-, 3,3'-Dimethoxy, 2'-Chlor-3,4-dimethoxy-, 3,4,5,3',4',5'-Hexamethoxy-benzil,
- Isatinderivate, wie Isatin-5-sulfonsäure, Isatin-4-carbonsäure und lsatin-5-carbonsäure,
- N-substituierte Isatin-Derivate, wie N-(2'-Hydroxyethyl)-isatin, N-(2'-Hydroxypropyl)-isatin, N-(3'-Hydroxypropyl)-isatin, N-(2',3'-Dihydroxypropyl)-isatin, N-(2'-Sulfoethyl)-isatin, (3'-Sulfopropyl)-isatin, N-Allylisatin, N-Vinylisatin, N-Benzylisatin, N-(4'-Methoxybenzyl)-isatin, N-(4'-Carboxybenzyl)-isatin, N-(4-Sulfobenzyl)-isatin, N-(2'-Dimethylaminoethyl)-isatin, N-(2'-Pyrrolidinoethyl)-isatin, N-(2'-Piperidinoethyl)-isatin, N-(2'-Morpholinoethyl)-isatin, N-(2-Furylmethyl)-isatin, N-(2-Thienylmethyl)-isatin, N-(2-Pyridylmethyl)-isatin, N-(3-Pyridylmethyl)-isatin, N-(4-Pyridylmethyl)-isatin, N-Allylisatin-5-sulfonsäure, 5-Chlor-N-(2'-hydroxyethyl)-isatin, 5-Methyl-N-(2'-hydroxyethyl)-isatin, 5,7-Dichlor-N-allylisatin, 5-Nitro-N-allylisatin, N-Hydroxymethylisatin, N-Hydroxymethyl-5-methylisatin, N-Hydroxymethyl-5-chlorisatin, N-Hydroxymethyl-5-sulfoisatin, N-Hydroxymethyl-5-carboxyisatin, N-Hydroxymethyl-5-nitroisatin, N-Hydroxymethyl-5-bromisatin, N-Hydroxymethyl-5-methoxyisatin, N-Hydroxymethyl-5,7-dichlorisatin, N-Dimethylaminomethylisatin, N-Diethylaminomethylisatin, N-(Bis-(2'-hydroxyethyl)-aminomethyl)-isatin, N-(2'-Hydroxyethylaminomethyl)-isatin, N-(Bis-(2'-hydroxypropyl)-aminomethyl)-isatin, N-Pyrrolidinomethylisatin, N-Piperidinomethylisatin, N-Morpholinomethylisatin, N-(1,2,4-Triazolyl)-methylisatin, N-(1-lmidazolyl)-methylisatin, N-Carboxymethylaminomethylisatin, N-(2'-Carboxyethylaminomethyl)-isatin, N-(3'-Carboxypropylaminomethyl)-isatin, N-(Bis-(2'-hydroxyethyl)-aminomethyl)-5-methylisatin, N-Piperidinomethyl-5-chlorisatin, N-(2'-Sulfoethylamino)-isatin, sowie die Alkali- und gegebenenfalls Ammoniumsalze der sauren Verbindungen,
- Chinisatin und deren Derivate, wie N-Methylchinisatin,
- Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxyacetophenon, 3,5-Dimethoxy-4-hydroxy-acetophenon, 4-Amino-acetophenon, 4-Dimethylamino-acetophenon, 4-Morpholino-acetophenon, 4-Piperidino-acetophenon, 4-Imidazolino-acetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxy-benzophenon, 2-Amino-benzophenon, 4,4'-Dihydroxy-benzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Indanon, 9-Fluorenon, 3-Hydroxyfluorenon, Anthron, 1,8-Dihydroxyanthron,
- heterocyclische Carbonylverbindungen, wie 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1,3,3-Trimethyl-2-indolinyliden)-acetaldehyd, 1-Methylpyrrol-2-aldehyd, 1-Methyl-2-acetylpyrrol, 1-Pyridinaldehyd, 2-Pyridinaldehyd, 3-Pyridinaldehyd, 1-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5-Nitro-2-furyl)-acrolein, 3-(2-Furyl)-acrolein, Imidazol-2-aldehyd, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin,
- Indanon-Derivate, wie z. B. 1,2-Indandion, 2-Oximo-1-indanon, Indan-1,2,3-trion-2-oxim, 5-Methoxy-indan-1,2,3-trion-2-oxim, 2-Nitro-1,3-indandion,
- quarternierte Azafluorenone, wie die Trifluormethansulfonate, Benzolsulfonate, p-Toluolsulfonate, Methansulfonate, Perchlorate, Sulfate, Chloride, Bromide, lodide, Tetrachlorzinkate, Hexafluorphosphate Tetrafluorborate von 1-Methyl-5-oxo-indeno[1,2-b]pyridinium-(4-methyl-4-azonio-9-fluorenon), 1-Ethyl-5-oxo-indeno[1,2-b]pyridinium, 1-Benzyl-5-oxo-indeno[1,2-b]pyridinium, 2-Methyl-5-oxo-indeno[1,2-c]pyridinium, 2-Methyl-9-oxo-indeno[2,1-c]pyridinium, 1-Methyl-9-oxo-ideno[2,1-b]pyridinium,
sowie beliebige Gemische der voranstehenden Verbindungen eingesetzt werden.

Beispiele für bevorzugt eingesetzte Verbindungen mit der Formel II sind 4-Amino-1-nitrobenzol, 2-Amino-1-nitrobenzol, 1,4-Diamino-2-nitrobenzol, 1,2-Diamino-4-nitrobenzol, 1-Amino-2-methyl-6-nitrobenzol, 3-Amino-6-methylamino-2-nitro-pyridin (Azarot), Pikraminsäure, [8-[(4'-Amino-2'-nitrophenyl)azo]-7-hydroxy-naphth-2-yl]-trimethylammoniumchlorid, [8-((4'-Amino-3'-nitrophenyl)azo)-7-hydroxy-naphth-2-yl]-trimethylammoniumchlorid (Basic Brown 17), 1-Hydroxy-2-amino-4,6-dinitro-benzol, 1-Amino-2-nitro-4-[bis-(2'-hydroxyethyl)-amino]-benzol (HC Red Nr. 13), 1-Amino-2-(2'hydroxyethyl)-amino-5-nitro-benzol (HC Yellow Nr. 5), 4-Amino-2-nitro-diphenylamin (HC Red Nr. 1), 1-Amino-2-nitro-4-(2'-hydroxyethylamino)-benzol (HC Red Nr. 7), 1-Amino-4-(2-hydroxyethyl)amino-5-chlor-2-nitrobenzol, 2-Chlor-5-nitro-N-(2'-hydroxyethyl)-1,4-phenylendiamin, 1-(2'-Hydroxyethylamino)-2-nitro-4-amino-benzol (HC Red Nr. 3), 4-Amino-3-nitrophenol, 4-Amino-2-nitrophenol, 6-Nitro-o-toluidin, 1-Amino-3-methyl-4-(2-hydroxyethylamino)-6-nitrobenzol (HC Violet Nr. 1), 1-Amino-2-nitro-4-(2',3'-dihydroxypropylamino)-5-chlorobenzol (HC Red Nr. 10), 4-Amino-2-nitrodiphenylamin-2'carbonsäure, 2-(4'-Amino-2'-nitroanilino)-benzoesäure, 6-Nitro-2,5-diaminopyridin, 2-Amino-6-chloro-4-nitrophenol, 1-Amino-2-(3'-nitrophenylazo)-7-phenylazo-8-naphthol-3,6-disulfonsäure, Dinatriumsalz (Acid blue Nr. 29), 1-Amino-2-(2'-hydroxy-4'nitrophenylazo)-8-naphthol-3,6-disulfonsäure Dinatriumsalz (Palatinchrome green), 1-Amino-2-(3'-chlor-2'-hydroxy-5'-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2'-nitrobenzylidenamino)-benzol, 2-[2-(Diethylamino)-ethylamino]-5-nitroanilin, 3-Amino-4-hydroxy-5-nitrobenzolsulfonsäure (Hydrat), 3-Amino-3'-nitrobiphenyl, 3-Amino-4-nitro-acenaphthen, 2-Amino-1-nitronaphthalin, 5-Amino-6-nitrobenzo-1,3-dioxol, 2-Amino-6-nitrobenzothiazol und beliebige Gemische der voranstehenden Verbindungen.

Diese Substanzen sind zum großen Teil literaturbekannt oder im Handel erhältlich oder nach bekannten Syntheseverfahren herstellbar.

Die voranstehend genannten Verbindungen mit der Formel I und Formel II werden vorzugsweise in den erfindungsgemäßen Mitteln jeweils in einer Menge von 0,03 bis 65 mmol, insbesondere von 1 bis 40 mmol, bezogen auf 100 g des gesamten Färbemittels, verwendet. Das molare Verhältnis von Aldehyd oder Keton mit der Formel I und der Nitro-aminoverbindung mit der Formel II kann im Bereich von 0,5 bis 2,0 liegen, wobei vorzugsweise äquimolare Mengen eingesetzt werden.

Die erfindungsgemäße Kombination kann als direktziehendes Färbemittel oder in Gegenwart von üblichen Oxidationsfarbstoffvorprodukten, wie üblichen Kuppler- und Entwicklerkomponenten, die bereits im einleitenden Teil der Beschreibung genannt wurden, eingesetzt werden.

Färbemittel, die als färbende Komponente die erfindungsgemäße Kombination allein enthalten, werden bevorzugt für Färbungen im gelb-, gelbbraun-, orange-, braunorange-, rot-, rotbraun- und violett-Bereich bis hin zu blauviolett und schwarz eingesetzt.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

In allen Färbemitteln können auch mehrere verschiedene Kombinationen aus den Verbindungen mit den Formeln I und II gemeinsam zum Einsatz kommen; ebenso können auch mehrere verschiedene Farbverstärker gemeinsam verwendet werden.

Auf die Anwesenheit von Oxidationsmitteln, z. B. H₂O₂, kann verzichtet werden. Es kann jedoch u. U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red BN, Basic Red 76, HC Blue 2, Disperse Blue 3, Basic Blue 99, HC Violet 1, Disperse Violet 1, Disperse Violet 4, Disperse Black 9 und Basic Brown 16 bekannten Verbindungen sowie 6-Nitro-1,2,3,4-tetrahydrochinoxalin, 4-N-Ethyl-1,4-bis(2'-hydroxyethylamino)-2-nitrobenzol-hydrochlorid und 1-Methyl-3-nitro-4-(2'-hydroxyethylamino)-benzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, daß die erfindungsgemäß enthaltenen Verbindungen der Formeln I und II oder die fakultativ enthaltenen Farbverstärker und direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Citronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise VinylpyrrolidonNinylacrylat-Copolymere, Polyvinylpyrrolidon und VinylpyrrolidonNinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/lsobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.
- weitere Oxidationsfarbstoffvorprodukte vom Entwickler und/oder Kupplertyp, wie sie beispielsweise in der WO 01/41716 offenbart werden, auf die hier ausdrücklich Bezug genommen wird

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65 mmol, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 12, vorzugsweise zwischen 4 und 10.

Ein weiterer Gegenstand der vorliegenden Erfindung betrifft die Verwendung von einer Kombination aus Aldehyden bzw. Ketonen mit der Formel I, 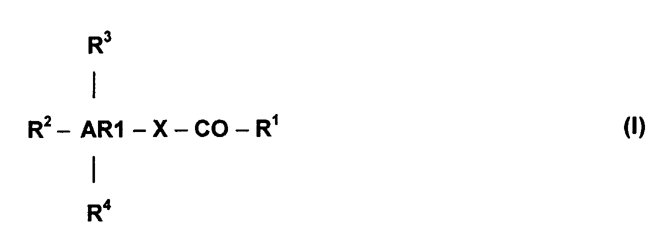 in der AR1, R¹, R², R³, R⁴ und X wie oben definiert sind,
und Nitro-Amino-Verbindungen mit der Formel II 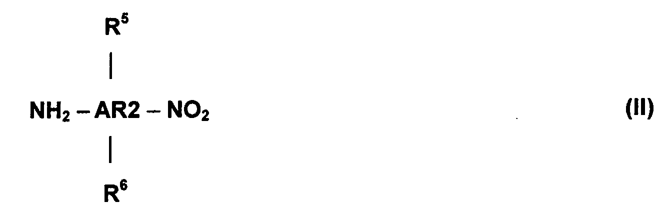 in der AR2, R⁵ und R⁶ wie oben definiert sind,
und/oder von Reaktionsprodukten aus den Verbindungen mit der Formel I und der Formel II als eine färbende Komponente in direktziehenden Färbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend eine Kombination aus Aldehyden bzw. Ketonen mit der Formel I, 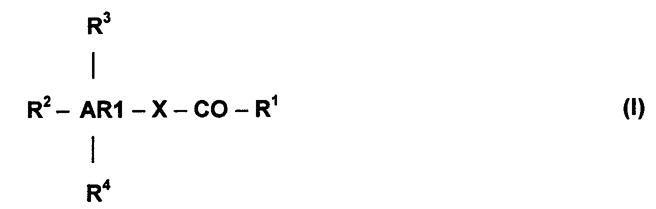 in der AR1, R¹, R², R³, R⁴ und X wie oben definiert sind,
und Verbindungen mit der Formeln II. 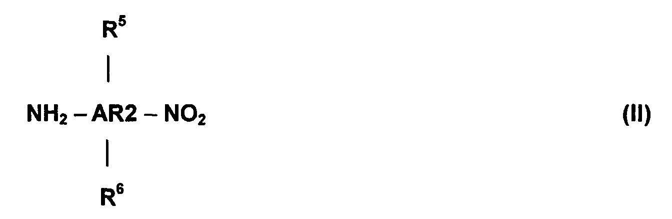 in der AR2, R⁵ und R⁶ wie oben definiert sind,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Aldehyde bzw. Ketone der Formel I und die Nitro-amino-Verbindungen mit der Formel II können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Aldehyde beziehungsweise Ketone der Formel I und die Nitro-amino-Verbindungen mit der Formel II können entweder getrennt oder zusammen gelagert werden, entweder in einer flüssigen bis pastösen Zubereitung (wäßrig oder wasserfrei) oder als trockenes Pulver. Bei der getrennten Lagerung werden die Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Herstellung einer Färbelösung

Es wurde je eine Aufschlämmung von 5 mmol eines Aldehyds oder Ketons der Formel I (A), 5 mmol einer Nitro-amino-Verbindung mit der Formel II (B) in 25 ml Wasser bei 50°C hergestellt. Die Aufschlämmungen wurden nach Abkühlen auf 30°C miteinander vermischt, mit 5 mmol Natriumacetat, ggf. 5 mmol Farbverstärker und einem Tropfen einer 20-%igen Fettalkylethersulfat-Lösung versetzt und mit verdünnter NaOH oder Salzsäure der pH-Wert entsprechend eingestellt.

In diese Färbemischung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft getrocknet und anschließend ausgekämmt.

Folgende Verbindungen mit der Formel I wurden als Verbindungen A eingesetzt:
- A1: 4-Dimethylaminobenzaldehyd
- A2: 2-Formylmethylen-1,3,3-trimethylindolin, (Tribasenaldehyd)
- A3: 4-Hydroxy-3-methoxyzimtaldehyd (Coniferylaldehyd)
- A4: 4-Hydroxynaphthaldehyd
- A5: 1-Methylindol-3-carbaldehyd
- A6: 4-Dimethylaminozimtaldehyd
- A7: 4-Dimethylamino-1-naphthaldehyd
- A8: 2,4-Dinitro-benzaldehyd
- A9: 3-Hydroxy-4-vitro-benzaldehyd

Folgende Verbindungen mit der Formel II wurden als Verbindungen B eingesetzt
- B1: 4-Amino-3-nitrophenol
- B2: [8-[(4'-Amino-3'-nitrophenyl)azo]-7-hydroxy-naphth-2-yl]-trimethylammoniumchlorid (Basic brown 17)
- B3: Pikraminsäure
- B4: 1-Amino-2-nitro-4-[bis-(2'-hydroxyethyl)-amino]-benzol; HC Red Nr. 13 (Kardinalrotpaste)
- B5: 1-(2'-Hydroxyethylamino)-2-nitro-4-aminobenzol, (HC Red Nr. 3)
- B6: 1-Amino-2-(2'-hydroxyethyl)-amino-5-nitrobenzol (HC Yellow Nr. 5)
- B7: 2-Amino-6-nitrobenzothiazol
- B8: 2-Nitro-3-amino-6-methylamino-pyridin (Azarot)

Die jeweiligen Farbnuancen und Farbtiefen sind in der nachfolgenden Tabelle 1 wiedergegeben.

Die Farbtiefe wurde dabei nach folgender Skala bewertet:
- - :: keine oder eine sehr blasse Ausfärbung
- (+) :: schwache Intensität
- + :: mittlere Intensität
- +(+) :: mittlere bis starke Intensität
- ++ :: starke Intensität
- ++(+) :: starke bis sehr starke Intensität
- +++ :: sehr starke Intensität

**Tabelle 1**

| **A** | **B** | **Farbverstärker** | **Farbe** | **Intensität** | **pH** |
|---|---|---|---|---|---|
| A1 | B1 | - | neutralrot | +++ | 6,0 |
| A1 | B2 | - | dunkelorange/braun | ++(+) | 6,0 |
| A1 | B3 | - | braunrot | ++(+) | 6,0 |
| A1 | B4 | - | dunkelviolett | +++ | 6,0 |
| A1 | B8 | - | orange | ++ | 6,0 |
| A2 | B1 | - | neutralrot | ++(+) | 6,0 |
| A2 | B4 | - | schwarzviolett | +++ | 6,0 |
| A2 | B5 | - | orangerot | +++ | 6,0 |
| A2 | B6 | - | orangerot | ++(+) | 6,0 |
| A2 | B8 | - | violettrot | ++(+) | 6,0 |
| A3 | B4 | - | dunkelrotviolett | +++ | 6,0 |
| A3 | B4 | Piperidin | dunkelrotbraun | +++ | 9,0 |
| A3 | B5 | - | braunorange | +(+) | 6,0 |
| A3 | B6 | - | braunorange | ++ | 6,0 |
| A3 | B7 | Piperidin | gelborange | ++ | 9,0 |
| A3 | B8 | - | orange | ++ | 6,0 |
| A4 | B1 | Piperidin | neutralrot | +++ | 9,0 |
| A4 | B4 | Piperidin | dunkelbraun | +++ | 9,0 |
| A5 | B4 | - | schwarzviolett | +++ | 6,0 |
| A5 | B5 | - | dunkelviolett | +++ | 6,0 |
| A5 | B6 | - | orangerot | ++(+) | 6,0 |
| A6 | B1 | Piperidin | leuchtend rot | ++(+) | 9,0 |
| A6 | B8 | - | gelborange | ++ | 6,0 |
| A7 | B7 | Piperidin | hellgelb | + | 9,0 |
| A8 | B1 | - | dunkelorange | ++ | 6,0 |
| A8 | B1 | Piperidin | braunorange | ++(+) | 9,0 |
| A8 | B4 | - | dunkelbraunviolett | +++ | 6,0 |
| A8 | B6 | - | orangebraun | ++(+) | 6,0 |
| A8 | B8 | - | orange | ++(+) | 6,0 |
| A9 | B4 | - | dunkelbraun | +++ | 6,0 |
| A9 | B8 | - | orange | ++ | 6,0 |

## Patentansprüche

1. Mittel zum Färben von keratinhaltigen Fasern, insbesondere menschlichen Haaren, enthaltend als färbende Komponente eine Kombination aus Aldehyden bzw. Ketonen mit der Formel I, 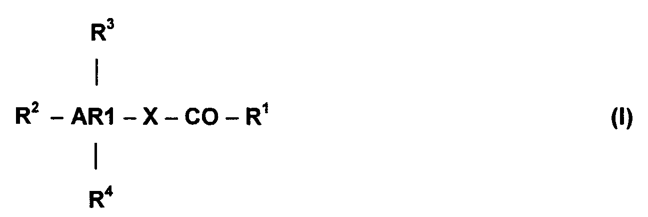 in der bedeuten
• AR1 Benzol, Naphthalin, Pyridin, Furan, Chinolin, Isochinolin, Chinolizin, Pyrimidin, Pyrrol, Thiophen, Indol, Indan, Imidazol, Benzimidazol, Benzothiazol, Indazol, Benzoxazol, Carbazol, Chinoxalin, Chinazolin, Cinnolin, Acenaphthen, Fluoren, Biphenyl, Diphenylmethan, Benzophenon, Diphenylether, Azobenzol, Diphenylamin, Stilben sowie deren teilweise oder vollständig hydrierte Derivate,
• R¹ ein Wasserstoffatom, eine C₁₋₄-Alkyl-, C₂₋₄-Alkenyl-, eine ggf. substituierte Aryl- oder Heteroarylgruppe,
• R², R³ und R⁴ unabhängig voneinander ein Wasserstoff-, ein Halogenatom, eine C₁₋₄-Alkyl-, C₁₋₄-Alkoxy-, C₁₋₄-Acyl-, eine C₁₋₄-Hydroxyalkyl-, eine Hydroxy-C₁₋₄alkoxy-, eine Hydroxy-, eine Sulfo-, eine Carboxy-, eine Nitro-, eine Morpholino-, eine Pyrrolidino-, eine Amino-, C₁₋₄-Acylamino-, eine Ammonio- oder eine 1-lmidazoli(ni)ogruppe, wobei die letzten 4 Gruppen mit C₁₋₄-Alkyl-, C₁₋₄-Carboxyalkyl-, C₁₋₄-Hydroxyalkyl-, C₂₋₆-Oligohydroxyalkyl-, C₂₋₄-Alkenylgruppen, mit ggf. substituierten Benzylgruppen, mit Sulfo-(C₁₋₄)-alkyl- oder Heterozyklus-(C₁₋₄)-alkylgruppen substituiert sein können, und auch zwei der Gruppen ausgewählt aus R², R³, R⁴ und -X-CO-R¹ miteinander einen ankondensierten gesättigten oder ungesättigten, carbozyklischen oder heterozyklischen, gegebenenfalls substituierten 5-, 6- oder 7-Ring, der ebenfalls einen ankondensierten aromatischen, ggf. substituierten Ring tragen kann, sein können, wobei das System AR1 in Abhängigkeit von der Größe des Ringes weitere Substituenten tragen kann, die unabhängig voneinander für die gleichen Gruppen stehen können wie R², R³ und R⁴, und
• X eine direkte Bindung, eine Carbonylgruppe, eine gegebenenfalls substituierte Methylen, Vinylen-, 1,3-Butadien-1,4-diyl oder Phenylengruppe,
und/oder deren funktionellen Carbonylderivaten und/oder physiologisch verträglichen Salzen
und Nitro-amino-Verbindungen mit der Formel II, 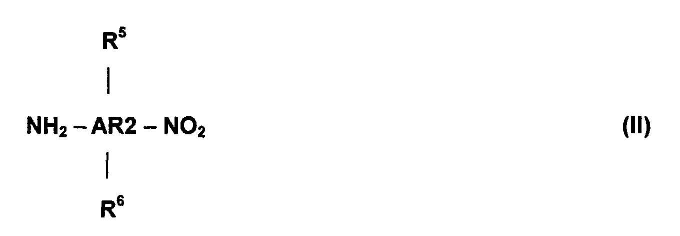 in der bedeuten
• AR2 Benzol, Naphthalin, Pyridin, Furan, Chinolin, Isochinolin, Pyrimidin, Pyrrol, Thiophen, Indol, Benzimidazol, Benzothiazol, Indazol, Benzoxazol, Chinoxalin, Chinazolin, Cinnolin, Acenaphthen, Fluoren, Biphenyl, Diphenylmethan, Benzophenon, Azobenzol, (Naphthyl)phenyldiazen, Diphenylamin, Stilben oder Benzylidenaminobenzol und
• R⁵ und R⁶ unabhängig voneinander ein Wasserstoffatom, Halogenatom, eine C₁₋₄-Alkyl-, C₁₋₄-Alkoxy-, C₁₋₄-Hydroxyalkoxy-, Hydroxy-, Nitro-, Carboxy-, C₁₋₄-Alkoxycarbonyl-, Sulfo-, Sulfamoyl-, eine Aryl- oder Heteroarylgruppe, Arylazogruppe oder eine Aminogruppe, die durch eine oder mehrere C₁₋₄-Alkyloder C₁₋₄-Hydroxyalkylgruppen substituiert sein kann,
wobei das System AR2 in Abhängigkeit von der Größe des Ringes weitere Substituenten tragen kann, die unabhängig voneinander für die gleichen Gruppen stehen können wie R⁵ und R⁶, und zwei der Reste aus R⁵, R⁶ und den ggf. vorhandenen weiteren Resten einen ankondensierten gesättigten oder ungesättigten, carbozyklischen oder heterozyklischen gegebenenfalls substituierten 5-, 6- oder 7-Ring, der ebenfalls einen ankondensierten aromatischen, ggf. substituierten Ring tragen kann, bilden können,
und/oder deren physiologisch verträglichen Salzen,
und/oder Reaktionsprodukte aus den Verbindungen mit der Formel I und der Formel II.

2. Mittel nach Anspruch 1, **dadurch gekennzeichnet, daß** als Verbindungen mit der Formel I
- 5-(4'-Dimethylaminophenyl)-penta-2,4-dienal, 5-(4'-Diethylaminophenyl)-penta-2,4-dienal, 5-(4'-Methoxyphenyl)-penta-2,4-dienal, 5-(3',4'-Dimethoxyphenyl)-penta-2,4-dienal, 5-(2',4'-Dimethoxyphenyl)-penta-2,4-dienal, 5-(4'-Piperidinophenyl)-penta-2,4-dienal, 5-(4'-Morpholinophenyl)-penta-2,4-dienal, 5-(4'-Pyrrolidinophenyl)-penta-2,4-dienal, 6-(4'-Dimethylaminophenyl)-hexa-2,4-dien-2-on, 6-(4'-Diethylaminophenyl)-hexa-2,4-dien-2-on, 6-(4'-Methoxyphenyl)-hexa-2,4-dien-2-on, 6-(3',4'-Dimethoxyphenyl)-hexa-2,4-dien-2-on, 6-(2',4'-Dimethoxyphenyl)-hexa-2,4-dien-2-on, 6-(4'-Piperidinophenyl)-hexa-2,4-dien-2-on, 6-(4'-Morpholinophenyl)-hexa-2,4-dien-2-on, 6-(4'-Pyrrolidinophenyl)-hexa-2,4-dien-2-on, 5-(4'-Dimethylamino-1'-naphthyl)-penta-2,4-dienal,
- 2-Nitro-piperonal, 5-Nitro-piperonal, 6-Nitro-piperonal, 5-Hydroxy-2-nitropiperonal, 2-Hydroxy-5-nitro-piperonal, 2-Chlor-6-nitro-piperonal, 5-Chlor-2-nitropiperonal, 2,6-Dinitro-piperonal,
- 2-Nitro-benzaldehyd, 3-Nitro-benzaldehyd, 4-Nitro-benzaldehyd, 6-Nitro-benzaldehyd, 4-Methyl-3-nitro-benzaldehyd, 3-Hydroxy-4-nitro-benzaldehyd, 4-Hydroxy-3-nitro-benzaldehyd, 5-Hydroxy-2-nitro-benzaldehyd, 2-Hydroxy-5-nitro-benzaldehyd, 2-Hydroxy-3-nitro-benzaldehyd, 2-Fluor-3-nitro-benzaldehyd, 3-Methoxy-2-nitro-benzaldehyd, 4-Chlor-3-nitro-benzaldehyd, 2-Chlor-6-nitro-benzaldehyd, 5-Chlor-2-nitro-benzaldehyd, 4-Chlor-2-nitro-benzaldehyd, 2,4-Dinitro-benzaldehyd, 2,6-Dinitro-benzaldehyd, 2-Hydroxy-3-methoxy-5-nitrobenzaldehyd, 4,5-Dimethoxy-2-nitro-benzaldehyd, 5-Nitrovanillin, 3,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd,
- Carbazolaldehyde oder Carbazolketone, insbesondere 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
- 4-Trimethylammoniobenzaldehyd-, 4-Benzyldimethylammoniobenzaldehyd-, 4-Trimethylammoniozimtaldehyd-, 4-Trimethylammonionaphthaldehyd-, 2-Methoxy-4-trimethylammoniobenzaldehyd-, N-(4'-Acetylphenyl)-trimethylammonium-, 4-(N,N-Diethyl)-N-methylammonio)-benzaldehyd-, N-(4'-Benzoylphenyl)-trimethylammonium-, N-(4-Benzoylphenyl)-N,N-diethylmethylammonium-, N-(4'-Formylphenyl)-N-methylpyrrolidinium-, N-(4'-Formylphenyl)-N-methylpiperidinium-, N-(4-Formylphenyl)-N-methylmorpholinium- N-(4'-Acetylphenyl)-N-methylmorpholinium-, N-(4'-Benzoylphenyl)-N-methylmorpholinium-, 3-Formyl-N-ethyl-N-methylcarbazolium-, 3-Formyl-9,9-dimethylcarbazolium-, 1-(4'-Acetylphenyl)-3-methylimidazolium-, 1-(4'-Acetylphenyl)-3-methyl-2-imidazolinium-, 1-(4'-Benzoylphenyl)-3-methylimidazolium-, 5-Acetyl-1,3-diethyl-2-methylbenzimidazolium-, 5-Trimethylammonio-1-indanon-Salze, insbesondere die Benzolsulfonate, p-Toluolfulfonate, Methansulfonate, Ethansulfonate, Propansulfonate, Perchlorate, Sulfate, Chloride, Bromide, lodide, Tetrachlorzinkate, Methylsulfate, Trifluormethansulfonate, Hexafluorphosphate, Tetrafluorborate,
- Salicylaldehyd, Vanillin, 4-Hydroxy-3-methoxyzimtaldehyd (Coniferylaldehyd), 2,4-Dihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 4-Piperidinobenzaldehyd, 4-Dimethylaminoacetophenon, 4-Hydroxynaphthaldehyd, 4-Dimethylaminonaphthaldehyd, 4-Dimethylaminobenzylidenaceton, 4-Dimethylaminozimtaldehyd, 2-Dimethylaminobenzaldehyd, 2-Chlor-4-dimethylaminobenzaldehyd, 4-Dimethylamino-2-methylbenzaldehyd, trans-4-Diethylamino-zimtaldehyd, 4-(Dibutylamino)-benzaldehyd, 4-Diphenylaminobenzaldehyd, 2,3,6,7-Tetrahydro-1H,5Hbenzo[ij]chinolizin-9-carboxaldehyd, 4-Dimethylamino-2-methoxybenzaldehyd, 2,3,6,7-Tetrahydro-8-hydroxy-1H,5H-benzo[ij]chinolizin-9-carboxaldehyd, 4-(1-lmidazolyl)-benzaldehyd, 2-Morpholinobenzaldehyd, Indol-3-carboxaldehyd, 1-Methylindol-3-carboxaldehyd, N-Ethylcarbazol-3-carboxaldehyd, 2-Formylmethylen-1,3,3-trimethylindolin (Tribasen Aldehyd)
- 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon, 2-(3'-Pyridoyl)-acetophenon,
- 1-Phenyl-1,2-propandion, 1-Phenyl-1,2-butandion, 1-(Chlorphenyl)-1,2-propandion, 1-Phenyl-3,3-dimethyl-1,2-butandion, Benzil, Anisil, Salicil, 5,5'-Dibromsalicil, 2,2'-Furyl, 2,2'-Thienyl, 2,2'-, 4,4'-Pyridil, 6,6'-Dimethyl-4,4'-pyridil, 4-Hydroxy-, 4-Methoxy-, 4-Chlor-, 4-Methyl-, 4-Dimethylamino-, 4,4'-Dihydroxy-,-Dimethyl-, -Dibrom-, -Dichlor-, -Bis-dimethylamino-, 2,4-Dihydroxy-, 3,3'-Dimethoxy, 2'-Chlor-3,4-dimethoxy-, 3,4,5,3',4',5'-Hexamethoxy-benzil,
- Isatinderivate, wie Isatin-5-sulfonsäure, Isatin-4-carbonsäure und Isatin-5-carbonsäure,
- N-substituierte Isatin-Derivate, wie N-(2'-Hydroxyethyl)-isatin, N-(2'-Hydroxypropyl)-isatin, N-(3'-Hydroxypropyl)-isatin, N-(2',3'-Dihydroxypropyl)-isatin, N-(2'-Sulfoethyl)-isatin, (3'-Sulfopropyl)-isatin, N-Allylisatin, N-Vinylisatin, N-Benzylisatin, N-(4'-Methoxybenzyl)-isatin, N-(4'-Carboxybenzyl)-isatin, N-(4'-Sulfobenzyl)-isatin, N-(2'-Dimethylaminoethyl)-isatin, N-(2'-Pyrrolidinoethyl)-isatin, N-(2-Piperidinoethyl)-isatin, N-(2-Morpholinoethyl)-isatin, N-(2-Furylmethyl)-isatin, N-(2-Thienylmethyl)-isatin, N-(2-Pyridylmethyl)-isatin, N-(3-Pyridylmethyl)-isatin, N-(4-Pyridylmethyl)-isatin, N-Allylisatin-5-sulfonsäure, 5-Chlor-N-(2'hydroxyethyl)-isatin, 5-Methyl-N-(2'-hydroxyethyl)-isatin, 5,7-Dichlor-N-allylisatin, 5-Nitro-N-allylisatin, N-Hydroxymethylisatin, N-Hydroxymethyl-5-methylisatin, N-Hydroxymethyl-5-chlorisatin, N-Hydroxymethyl-5-sulfoisatin, N-Hydroxymethyl-5-carboxyisatin, N-Hydroxymethyl-5-nitroisatin, N-Hydroxymethyl-5-bromisatin, N-Hydroxymethyl-5-methoxyisatin, N-Hydroxymethyl-5,7-dichlorisatin, N-Dimethylaminomethylisatin, N-Diethylaminomethylisatin, N-(Bis-(2'-hydroxyethyl)-aminomethyl)-isatin, N-(2'-Hydroxyethylaminomethyl)-isatin, N-(Bis-(2'-hydroxypropyl)-aminomethyl)-isatin, N-Pyrrolidinomethylisatin, N-Piperidinomethylisatin, N-Morpholinomethylisatin, N-(1,2,4-Triazolyl)-methylisatin, N-(1-Imidazolyl)-methylisatin, N-Carboxymethylaminomethylisatin, N-(2'-Carboxyethylaminomethyl)-isatin, N-(3'-Carboxypropylaminomethyl)-isatin, N-(Bis-(2'-hydroxyethyl)-aminomethyl)-5-methylisatin, N-Piperidinomethyl-5-chlorisatin, N-(2'-Sulfoethylamino)-isatin, sowie die Alkali- und gegebenenfalls Ammoniumsalze der sauren Verbindungen,
- Chinisatin und deren Derivate, wie N-Methylchinisatin,
- Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxy-acetophenon, 3,5-Dimethoxy-4-hydroxy-acetophenon, 4-Amino-acetophenon, 4-Dimethylamino-acetophenon, 4-Morpholino-acetophenon, 4-Piperidino-acetophenon, 4-Imidazolino-acetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxy-benzophenon, 2-Amino-benzophenon, 4,4'-Dihydroxy-benzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Indanon, 9-Fluorenon, 3-Hydroxyfluorenon, Anthron, 1,8-Dihydroxyanthron,
- heterocyclische Carbonylverbindungen, wie 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1,3,3-Trimethyl-2-indolinyliden)-acetaldehyd, 1-Methylpyrrol-2-aldehyd, 1-Methyl-2-acetylpyrrol, 1-Pyridinaldehyd, 2-Pyridinaldehyd, 3-Pyridinaldehyd, 1-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5-Nitro-2-furyl)-acrolein, 3-(2-Furyl)-acrolein, Imidazol-2-aldehyd, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin,
- Indanon-Derivate, wie z. B. 1,2-Indandion, 2-Oximo-1-indanon, Indan-1,2,3-trion-2-oxim, 5-Methoxy-indan-1,2,3-trion-2-oxim, 2-Nitro-1,3-indandion,
- quarternierte Azafluorenone, wie die Trifluormethansulfonate, Benzolsulfonate, p-Toluolsulfonate, Methansulfonate, Perchlorate, Sulfate, Chloride, Bromide, lodide, Tetrachlorzinkate, Hexafluorphosphate Tetrafluorborate von 1-Methyl-5-oxo-indeno[1,2-b]pyridinium-(4-methyl-4-azonio-9-fluorenon), 1-Ethyl-5-oxo-indeno[1,2-b]pyridinium, 1-Benzyl-5-oxo-indeno[1,2-b]pyridinium, 2-Methyl-5-oxoindeno[1,2-c]pyridinium, 2-Methyl-9-oxo-indeno[2,1-c]pyridinium, 1-Methyl-9-oxoideno[2,1-b]pyridinium,
sowie beliebige Gemische der voranstehenden Verbindungen eingesetzt werden.

3. Mittel nach Anspruch 1 oder 2, **dadurch gekennzeichnet, daß** als Nitro-amino-verbindungen mit der Formel II 4-Amino-1-nitrobenzol, 2-Amino-1-nitrobenzol, 1,4-Diamino-2-nitrobenzol, 1,2-Diamino-4-nitrobenzol, 1-Amino-2-methyl-6-nitrobenzol, 3-Amino-6-methylamino-2-nitro-pyridin (Azarot), Pikraminsäure, [8-[(4'-Amino-2'nitrophenyl)azo]-7-hydroxy-naphth-2-yl]-trimethylammoniumchlorid, [8-((4'-Amino-3'nitrophenyl)azo)-7-hydroxy-naphth-2-yl]-trimethylammoniumchlorid (Basic Brown 17), 1-Hydroxy-2-amino-4,6-dinitro-benzol, 1-Amino-2-nitro-4-[bis-(2'-hydroxyethyl)-amino]-benzol (HC Red Nr. 13), 1-Amino-2-(2'-hydroxyethyl)-amino-5-nitro-benzol (HC Yellow Nr. 5), 4-Amino-2-nitro-diphenylamin (HC Red Nr. 1), 1-Amino-2-nitro-4-(2'-hydroxyethylamino)-benzol (HC Red Nr. 7), 1-Amino-4-(2-hydroxyethyl)amino-5-chlor-2-nitrobenzol, 2-Chlor-5-nitro-N-(2'-hydroxyethyl)-1,4-phenylendiamin, 1-(2'-Hydroxyethylamino)-2-nitro-4-amino-benzol (HC Red Nr. 3), 4-Amino-3-nitrophenol, 4-Amino-2-nitrophenol, 6-Nitro-o-toluidin, 1-Amino-3-methyl-4-(2-hydroxyethylamino)-6-nitrobenzol (HC Violet Nr. 1), 1-Amino-2-nitro-4-(2',3'-dihydroxypropylamino)-5-chlorobenzol (HC Red Nr. 10), 4-Amino-2-nitrodiphenylamin-2'carbonsäure, 2-(4'-Amino-2'-nitroanilino)-benzoesäure, 6-Nitro-2,5-diaminopyridin, 2-Amino-6-chloro-4-nitrophenol, 1-Amino-2-(3'-nitrophenylazo)-7-phenylazo-8-naphthol-3,6-disulfonsäure, Dinatriumsalz (Acid blue Nr. 29), 1-Amino-2-(2'-hydroxy-4'-nitrophenylazo)-8-naphthol-3,6-disulfonsäure Dinatriumsalz (Palatinchrome green), 1-Amino-2-(3'-chlor-2'-hydroxy-5'-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2'-nitrobenzylidenamino)-benzol, 2-[2-(Diethylamino)-ethylamino]-5-nitroanilin, 3-Amino-4-hydroxy-5-nitrobenzolsulfonsäure (Hydrat), 3-Amino-3'-nitrobiphenyl, 3-Amino-4-nitroacenaphthen, 2-Amino-1-nitronaphthalin, 5-Amino-6-nitrobenzo-1,3-dioxol, 2-Amino-6-nitrobenzothiazol und beliebige Gemische der voranstehenden Verbindungen.

4. Mittel nach einem der Ansprüche 1 bis 3, **dadurch gekennzeichnet, daß** die Aldehyde bzw. Ketone mit der Formel I und Nitro-amino-verbindungen mit der Formel II jeweils in einer Menge von 0,03 bis 65 mmol, insbesondere von 1 bis 40 mmol, bezogen auf 100 g des gesamten Färbemittels, enthalten sind.

5. Mittel nach einem der Ansprüche 1 bis 4, **dadurch gekennzeichnet, daß** der Aldehyd bzw. das Keton mit der Formel I und die Nitro-aminoverbindung der Formel II im molaren Verhältnis von 0,5 bis 2,0 liegen.

6. Mittel nach einem der Ansprüche 1 bis 5, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazin oder deren beliebigen Gemischen enthält.

7. Mittel nach einem der Ansprüche 1 bis 6, **dadurch gekennzeichnet, daß** es direktziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

8. Mittel nach einem der Ansprüche 1 bis 7, **dadurch gekennzeichnet, daß** es anionische, zwitterionische oder nichtionische Tenside enthält.

9. Mittel nach einem der Ansprüche 1 bis 8, **dadurch gekennzeichnet, daß** es Oxidationsmittel, insbesondere H₂O₂, in einer Menge von 0,01 bis 6 Gew.-%, bezogen auf die Anwendungslösung, enthält.

10. Mittel nach einem der Ansprüche 1 bis 9, **dadurch gekennzeichnet, daß** es Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, enthält.

11. Verwendung von einer Kombination aus Aldehyden bzw. Ketonen mit der Formel I, 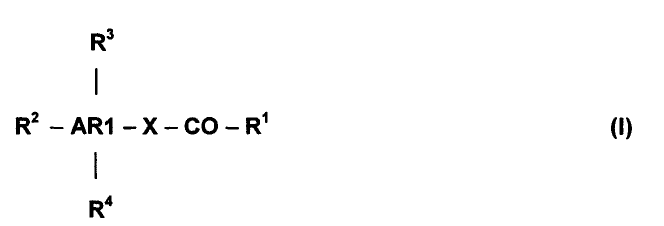 in der bedeuten
• AR1 Benzol, Naphthalin, Pyridin, Furan, Chinolin, Isochinolin, Chinolizin, Pyrimidin, Pyrrol, Thiophen, Indol, Indan, Imidazol, Benzimidazol, Benzothiazol, Indazol, Benzoxazol, Carbazol, Chinoxalin, Chinazolin, Cinnolin, Acenaphthen, Fluoren, Biphenyl, Diphenylmethan, Benzophenon, Diphenylether, Azobenzol, Diphenylamin, Stilben sowie deren teilweise oder vollständig hydrierte Derivate,
• R¹ ein Wasserstoffatom, eine C₁₋₄-Alkyl-, C₂₋₄-Alkenyl-, eine ggf. substituierte Aryl- oder Heteroarylgruppe,
• R², R³ und R⁴ unabhängig voneinander ein Wasserstoff-, ein Halogenatom, eine C₁₋₄-Alkyl-, C₁₋₄-Alkoxy-, C₁₋₄-Acyl-, eine C₁₋₄-Hydroxyalkyl-, eine Hydroxy-C₁₋₄alkoxy-, eine Hydroxy-, eine Sulfo-, eine Carboxy-, eine Nitro-, eine Morpholino-, eine Pyrrolidino-, eine Amino-, C₁₋₄-Acylamino-, eine Ammonio- oder eine 1-lmidazoli(ni)ogruppe, wobei die letzten 4 Gruppen mit C₁₋₄-Alkyl-, C₁₋₄-Carboxyalkyl-, C₁₋₄-Hydroxyalkyl-, C₂₋₆-Oligohydroxyalkyl-, C₂₋₄-Alkenylgruppen, mit ggf. substituierten Benzylgruppen, mit Sulfo-(C₁₋₄)-alkyl- oder Heterozyklus-(C₁₋₄)-alkylgruppen substituiert sein können, und auch zwei der Gruppen ausgewählt aus R², R³, R⁴ und -X-CO-R¹ miteinander einen ankondensierten gesättigten oder ungesättigten, carbozyklischen oder heterozyklischen, gegebenenfalls substituierten 5-, 6- oder 7-Ring, der ebenfalls einen ankondensierten aromatischen, ggf. substituierten Ring tragen kann, sein können, wobei das System AR1 in Abhängigkeit von der Größe des Ringes weitere Substituenten tragen kann, die unabhängig voneinander für die gleichen Gruppen stehen können wie R², R³ und R⁴, und
• X eine direkte Bindung, eine Carbonylgruppe, eine gegebenenfalls substituierte Methylen, Vinylen-, 1,3-Butadien-1,4-diyl oder Phenylengruppe,
und/oder deren funktionellen Carbonylderivaten und/oder physiologisch verträglichen Salzen
und Nitro-amino-Verbindungen mit der Formel II, 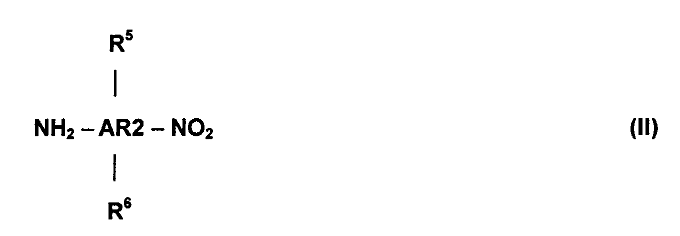 in der bedeuten
• AR2 Benzol, Naphthalin, Pyridin, Furan, Chinolin, Isochinolin, Pyrimidin, Pyrrol, Thiophen, Indol, Benzimidazol, Benzothiazol, Indazol, Benzoxazol, Chinoxalin, Chinazolin, Cinnolin, Acenaphthen, Fluoren, Biphenyl, Diphenylmethan, Benzophenon, Azobenzol, (Naphthyl)phenyldiazen, Diphenylamin, Stilben oder Benzylidenaminobenzol und
• R⁵ und R⁶ unabhängig voneinander ein Wasserstoffatom, Halogenatom, eine C₁₋₄-Alkyl-, C₁₋₄-Alkoxy-, C₁₋₄-Hydroxyalkoxy-, Hydroxy-, Nitro-, Carboxy-, C₁₋₄-Alkoxycarbonyl-, Sulfo-, Sulfamoyl-, eine Aryl- oder Heteroarylgruppe, Arylazogruppe oder eine Aminogruppe, die durch eine oder mehrere C₁₋₄-Alkyloder C₁₋₄-Hydroxyalkylgruppen substituiert sein kann,
wobei das System AR2 in Abhängigkeit von der Größe des Ringes weitere Substituenten tragen kann, die unabhängig voneinander für die gleichen Gruppen stehen können wie R⁵ und R⁶, und zwei der Reste aus R⁵, R⁶ und den ggf. vorhandenen weiteren Resten einen ankondensierten gesättigten oder ungesättigten, carbozyklischen oder heterozyklischen gegebenenfalls substituierten 5-, 6- oder 7-Ring, der ebenfalls einen ankondensierten aromatischen, ggf. substituierten Ring tragen kann, bilden können,
und/oder deren physiologisch verträglichen Salzen,
und/oder Reaktionsprodukte aus den Verbindungen mit der Formel I und der Formel II.

12. Verfahren zum Färben von keratinhaltigen Fasern, insbesondere menschlichen Haaren, worin ein Färbemittel, enthaltend mindestens eine Kombination aus Aldehyden bzw. Ketonen mit der Formel I, 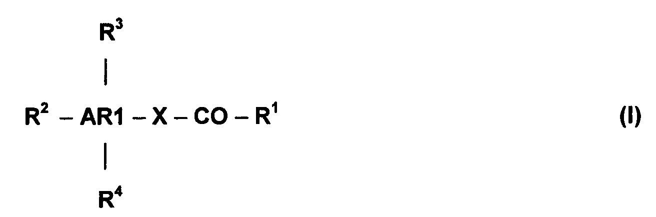 in der bedeuten
• AR1 Benzol, Naphthalin, Pyridin, Furan, Chinolin, Isochinolin, Chinolizin, Pyrimidin, Pyrrol, Thiophen, Indol, Indan, Imidazol, Benzimidazol, Benzothiazol, Indazol, Benzoxazol, Carbazol, Chinoxalin, Chinazolin, Cinnolin, Acenaphthen, Fluoren, Biphenyl, Diphenylmethan, Benzophenon, Diphenylether, Azobenzol, Diphenylamin, Stilben sowie deren teilweise oder vollständig hydrierte Derivate,
• R¹ ein Wasserstoffatom, eine C₁₋₄-Alkyl-, C₂₋₄-Alkenyl-, eine ggf. substituierte Aryl- oder Heteroarylgruppe,
• R², R³ und R⁴ unabhängig voneinander ein Wasserstoff-, ein Halogenatom, eine C₁₋₄-Alkyl-, C₁₋₄-Alkoxy-, C₁₋₄-Acyl-, eine C₁₋₄-Hydroxyalkyl-, eine Hydroxy-C₁₋₄alkoxy-, eine Hydroxy-, eine Sulfo-, eine Carboxy-, eine Nitro-, eine Morpholino-, eine Pyrrolidino-, eine Amino-, C₁₋₄-Acylamino-, eine Ammonio- oder eine 1-lmidazoli(ni)ogruppe, wobei die letzten 4 Gruppen mit C₁₋₄Alkyl-, C₁₋₄-Carboxyalkyl-, C₁₋₄-Hydroxyalkyl-, C₂₋₆-Oligohydroxyalkyl-, C₂₋₄-Alkenylgruppen, mit ggf. substituierten Benzylgruppen, mit Sulfo-(C₁₋₄)-alkyl- oder Heterozyklus-(C₁₋₄)-alkylgruppen substituiert sein können, und auch zwei der Gruppen ausgewählt aus R², R³, R⁴ und -X-CO-R¹ miteinander einen ankondensierten gesättigten oder ungesättigten, carbozyklischen oder heterozyklischen, gegebenenfalls substituierten 5-, 6- oder 7-Ring, der ebenfalls einen ankondensierten aromatischen, ggf. substituierten Ring tragen kann, sein können, wobei das System AR1 in Abhängigkeit von der Größe des Ringes weitere Substituenten tragen kann, die unabhängig voneinander für die gleichen Gruppen stehen können wie R², R³ und R⁴, und
• X eine direkte Bindung, eine Carbonylgruppe, eine gegebenenfalls substituierte Methylen, Vinylen-, 1,3-Butadien-1,4-diyl oder Phenylengruppe,
und/oder deren funktionellen Carbonylderivaten und/oder physiologisch verträglichen Salzen
und Nitro-amino-Verbindungen mit der Formel II, 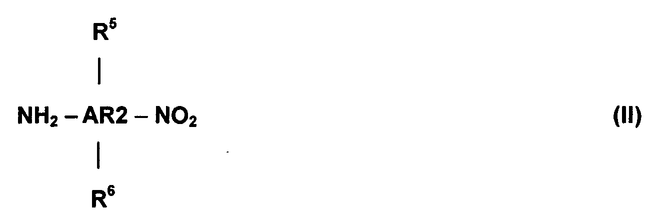 in der bedeuten
• AR2 Benzol, Naphthalin, Pyridin, Furan, Chinolin, Isochinolin, Pyrimidin, Pyrrol, Thiophen, Indol, Benzimidazol, Benzothiazol, Indazol, Benzoxazol, Chinoxalin, Chinazolin, Cinnolin, Acenaphthen, Fluoren, Biphenyl, Diphenylmethan, Benzophenon, Azobenzol, (Naphthyl)phenyldiazen, Diphenylamin, Stilben oder Benzylidenaminobenzol und
• R⁵ und R⁶ unabhängig voneinander ein Wasserstoffatom, Halogenatom, eine C₁₋₄-Alkyl-, C₁₋₄-Alkoxy-, C₁₋₄-Hydroxyalkoxy-, Hydroxy-, Nitro-, Carboxy-, C₁₋₄-Alkoxycarbonyl-, Sulfo-, Sulfamoyl-, eine Aryl- oder Heteroarylgruppe, Arylazogruppe oder eine Aminogruppe, die durch eine oder mehrere C₁₋₄-Alkyloder C₁₋₄-Hydroxyalkylgruppen substituiert sein kann,
wobei das System AR2 in Abhängigkeit von der Größe des Ringes weitere Substituenten tragen kann, die unabhängig voneinander für die gleichen Gruppen stehen können wie R⁵ und R⁶, und zwei der Reste aus R⁵, R⁶ und den ggf. vorhandenen weiteren Resten einen ankondensierten gesättigten oder ungesättigten, carbozyklischen oder heterozyklischen gegebenenfalls substituierten 5-, 6- oder 7-Ring, der ebenfalls einen ankondensierten aromatischen, ggf. substituierten Ring tragen kann, bilden können,
und/oder deren physiologisch verträglichen Salzen, und/oder Reaktionsprodukte aus den Verbindungen mit der Formel I und der Formel II sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.
